# EUROPÄISCHE PATENTSCHRIFT

(11) **EP 4 125 541 B1**
(45) Veröffentlichungstag und Bekanntmachung des Hinweises auf die Patenterteilung: **10.01.2024**
(21) Anmeldenummer: 21716136.3
(22) Anmeldetag: 29.03.2021
(51) Int. Cl.: A61B 1/012, A61B 1/018, A61B 1/04, A61F 2/95

(54) **ENDOSKOPIEREINRICHTUNG**
ENDOSCOPY EQUIPMENT
ÉQUIPEMENT ENDOSCOPIQUE

(30) Priorität: 30.03.2020 DE 102020108748
(43) Veröffentlichungstag der Anmeldung: 08.02.2023
(73) Patentinhaber: Freitag, Lutz, 58675 Hemer (DE)
(72) Erfinder: FREITAG, Prof. Dr. Med. Lutz, 58675 Hemer (DE); GEROLD, Manuel, 8302 Kloten (CH)
(74) Vertreter: Griepenstroh, Jörg
(86) Internationale Anmeldenummer: PCT/EP2021/058118
(87) Internationale Veröffentlichungsnummer: WO 2021/198159

(56) Entgegenhaltungen:
- US-A1- 2008 183 272
- US-A1- 2012 041 533
- US-A1- 2012 041 534
- US-A1- 2017 135 830

## Beschreibung

Die Erfindung betrifft eine Endoskopiereinrichtung mit den Merkmalen des Patentanspruchs 1.

Die Anwendung eines Endoskops wird für minimalinvasive operative Eingriffe an Menschen und Tieren sowie in der Technik, zur Sichtprüfung schwer zugänglicher Hohlräume, eingesetzt. Endoskope wurden ursprünglich für die humanmedizinische Diagnostik entwickelt. Endoskope werden auch zur Platzierung von Stents benutzt. Üblicherweise wird hierzu ein äußeres Endoskop bis vor die Engstelle eingebracht, an der der Stent zu platzieren ist. Anschließend wird in das äußere Endoskop ein inneres Endoskop geschoben. Dieses innere Endoskop wird mit dem Stent beladen. Ist der Stent in der richtigen Position, wird das innere Endoskop zurückgezogen, sodass der Stent freigegeben wird. Ein starrer Pusher hält den Stent hierbei in Position, wenn das innere Endoskop zurückgezogen wird.

Es ist allerdings handwerklich schwierig, den Stent zu positionieren, gleichzeitig das innere Endoskop zurückzuziehen und auch noch das äußere Endoskop zu führen. In der Humanmedizin erfolgt das Platzieren von Stents oder allgemein von expandierbaren Körpern unter Röntgenkontrolle und verlangt vom Operateur großes Geschick.

Es sind Videosticks bekannt, die inzwischen auch relativ kleine Durchmesser haben. Es ist in der Bronchoskopie möglich, das Endoskop per Videokontrolle zunächst an die richtige Position zu bringen, bevor der Stent aus dem Endoskop geschoben wird. Allerdings muss zuvor der Videostick zurückgezogen werden und ein Pusher in das Endoskop eingeführt werden, um den Stent freizugeben. Eine Videooptik ist in der Regel eine starre Optik, die nicht dazu geeignet ist, Druckkräfte auszuüben.

Die US 2012/0041533 A1 offenbart eine Endoskopiereinrichtung zum Einbringen eines Stents in einen Hohlraum eines Körpers. Der Stent befindet sich hinter einer Videospitze in einer ringförmigen Vertiefung. Wenn die Video-Spitze mit der sich anschließenden ringförmigen Vertiefung vorgeschoben wird, oder das außenseitig umgebende Endoskop zurückgezogen wird, kann sich der Stent in den umgebenden Hohlraum entfalten.

Die US 2012/0041534 A1 offenbart eine weitere Ausführungsform einer Endoskopiereinrichtung zum Einbringen eines Stents, wobei mehrere Videosysteme vorgesehen sind. Eine Kamera befindet sich am distalen Ende. Proximal hinter der Kamera befindet sich ein ringförmig verjüngter Bereich, in welchem ein zu platzierender Stent angeordnet ist. Durch Zurückziehen einer den Stent umgebenden Hülle bzw. durch Vorschieben der distalen Videoeinheit kann der Stent freigesetzt werden. Anschließend wird die Videoeinheit zurückgezogen.

Die US 2017/135830 A1 offenbart eine Kamera an einem distalen Ende einer Endoskopiereinrichtung, wobei die Kamera von einem Stent umgeben ist. Durch einen hülsenförmigen Pusher wird der Stent von der innenliegenden Kamera heruntergeschoben, während das außenliegende Rohr zurückgezogen wird. Während der ganzen Zeit bleibt die Kamera am distalen Ende des äußeren Rohres ausgerichtet und wird nicht relativ zu den anderen Komponenten des Endoskopkopfes bewegt.

Der Erfindung liegt die Aufgabe zugrunde, eine Endoskopiereinrichtung zum Einbringen eines expandierbaren Körpers in einen Hohlraum aufzuzeigen, die das Platzieren eines expandierbaren Körpers in dem Hohlraum vereinfacht.

Eine Endoskopiereinrichtung mit den Merkmalen des Patentanspruchs 1 löst diese Aufgabe.

Die abhängigen Patentansprüche betreffen vorteilhafte Weiterbildungen der Erfindung.

Die erfindungsgemäße Endoskopiereinrichtung ist nicht auf Anwendungen in der Humanmedizin oder Tiermedizin beschränkt. Die Endoskopiereinrichtung kann für einen beliebigen Hohlraum im menschlichen oder tierischen Körper vorgesehen sein oder auch zur Anwendung in technischen Bereichen vorgesehen sein.

Die erfindungsgemäße Endoskopiereinrichtung eignet sich insbesondere für die Platzierungen von Stents in der Luftröhre oder Speiseröhre.

Die Endoskopiereinrichtung umfasst ein erstes Endoskop mit einem Arbeitskanal, in den ein weiteres Endoskop mit dem einzubringenden expandierbaren Körper einführbar ist. In dem weiteren Endoskop befindet sich parallel zu dem expandierbaren Körper eine Videoeinheit. Ein distales Ende der Videoeinheit befindet sich neben einem distalen Ende des expandierbaren Körpers, um den expandierbaren Körper unter visueller Kontrolle in den Hohlraum zu platzieren.

Die Videoeinheit wird nicht separat oder getrennt von dem expandierbaren Körper verwendet, sondern parallel zu dem expandierbaren Körper, d. h., sie ist örtlich parallel und zeitgleich in dem weiteren, inneren Endoskop angeordnet. Dadurch ist es möglich, mittels der Videoeinheit das distale Ende des expandierbaren Körpers unter Videokontrolle an der gewünschten Stelle, insbesondere hinter einer Engstelle im Bereich des Hohlraums, zu platzieren. Nun wird das Endoskop, in dem sich der expandierbare Körper befindet, in Position gehalten und die Videoeinheit als Widerlager genutzt.

Bei dem verwendeten Endoskop kann es sich insbesondere um ein Endoskoprohr aus einem Kunststoff mit geringer Reibung handeln, z.B. PTFE, oder um ein Endoskoprohr mit einer geeigneten inneren und/oder äußeren Beschichtung handeln, wobei die Beschichtung die Reibung mindert und insbesondere aus PTFE besteht. In das Endoskoprohr ist ein gefalteter Stent als expandierbarer Körper, z.B. ein Stent aus einem polymeren Werkstoff, einbrinbar, bzw. es befindet sich vor seiner Positionierung in dem Hohlraum ein expandierbarer Körper in dem Endoskoprohr.

Die Videoeinheit kann nach dem Positionieren des expandierbaren Körpers soweit zurückgezogen werden, bis sie an dem proximalen Ende des expandierbaren Körpers anliegt. Da die Videoeinheit die Möglichkeit der visuellen Kontrolle bietet, ist die exakte Position des distalen Endes der Videoeinheit leicht einzustellen. Nun kann das distale Ende der Videoeinheit selbst als Widerlager dienen. Bei dieser Vorgehensweise ist es möglich, eine einheitlich schlanke Videoeinheit zu verwenden, die keine unterschiedlichen Durchmesserbereiche aufweist. Es wird allerdings vorausgesetzt, dass der expandierbare Körper eine hinreichend große Stirnfläche hat, an der das Endoskop als Widerlager zur Anlage gelangen kann, ohne dass der expandierbare Körper abrutscht. In einer vorteilhaften Weiterbildung der Erfindung ist die Videoeinheit flexibel, wobei die Flexibilität vorzugsweise im Bereich ihres distalen Endes größer ist als an dem proximalen Ende. Eine begrenzte Flexibilität ist bei bestimmten Anwendungsfällen von Vorteil, insbesondere bei der Bronchoskopie.

Das Verfahren, welches nicht Teil der Erfindung ist, sieht vor, zunächst ein äußeres Endoskop in dem Hohlraum zu positionieren und anschließend ein inneres Endoskop mit der Videoeinheit einzuführen, wobei zuvor ein distales Ende der Videoeinheit neben dem distalen Ende des expandierbaren Körpers in dem inneren Endoskop platziert wird. Der Ausdruck "neben" bedeutet, dass die distalen Enden der Videoeinheit und des expandierbaren Körpers bündig angeordnet sind oder zumindest im Wesentlichen bündig angeordnet sind. Das innere Endoskop wird unter Videokontrolle mittels der Videoeinheit bis zur gewünschten Tiefe in dem Hohlraum platziert. Die Videokontrolle schließt nicht aus, dass zusätzlich eine Röntgenkontrolle erfolgt. Nach dem korrekten Platzieren des inneren Endoskops bzw. nach dem Platzieren des expandierbaren Körpers, ist dieser im nächsten Schritt zu expandieren. Hierzu wird der expandierbare Körper in der gewünschten Position gehalten und das ihn umhüllende Endoskop zurückgezogen. Hierzu bedarf es eines Widerlagers, um die Reibung zwischen dem expandierbaren Körper und der Wand des Arbeitskanals, an dem der expandierbare Körper anliegt, zu überwinden. Als Widerlager dient die Videoeinheit selbst, indem die Videoeinheit zurückgezogen und am proximalen Ende des expandierbaren Körpers platziert wird. Nun kann das Endoskop, das den expandierbaren Körper bevorzugt und nur durch Haftreibung hält, zurückgezogen werden.

Unter "Expandieren" ist auch das bloße Entfalten/Entrollen, z.B. eines Silikons-Stents zu verstehen, also der Übergang von der im Durchmesser kleineren Transportposition innerhalb des Endoskoprohrs in eine Freigabeposition außerhalb des Endoskoprohrs. Bei selbstexpandierbaren Metallstents bedeutet das Expandieren/Freigeben einen Durchmesserzuwachs in Anpassung an den Hohlraum.

Eine Weiterentwicklung sieht ferner vor, dass ein weiteres Endoskop als Widerlager am proximalen Ende des expandierbaren Körpers platziert wird. Hierbei handelt es sich insbesondere um ein Endoskop/Instrument, das eine Greifvorrichtung an seinem distalen Ende aufweist. Dadurch kann die Lage des expandierbaren Körpers in Bezug auf seine Längsachse korrigiert werden, zum Beispiel wenn der expandierbare Körper eine abgeflachte Seite besitzt, die in einem entsprechenden Bereich des Hohlraums zur Anlage gelangen soll. Eine Greifvorrichtung im Sinne der Erfindung kann einen feststehenden oder betätigbaren Greifer aufweisen. Im einfacheren Fall ist die Greifvorrichtung eine Kupplung, wie z.B. bei einer Nut-FederAnordnung. Die Greifvorrichtung besitzt eine Nut, die auf den stirnseitigen, proximalen Rand des expandierbaren Körpers klemmend oder mit Spiel gesteckt werden kann, z.B um den expandierbaren Körper zu drehen. Ein betätigbarer Greifer kann wie eine Zange mit einem beweglichen Zangenkopf aufgebaut sein. Die Videoeinheit kann zudem eine längsseitige Nut aufweisen, die als Kanal zum Durchleiten von Gas vom proximalen zum distalen Ende dient. Der Kanal dient bei Anwendungen im Bereich der Bronchoskopie zur Sauerstoffversorgung des Patienten.

Die Erfindung wird nachfolgend anhand von rein schematischen Zeichnungen und dargestellten Ausführungsbeispielen erläutert. Es zeigen:
- Figur 1: eine Seitenansicht der Endoskopiereinrichtung in einer ersten Ausführungsform;
- Figur 2: eine Stirnansicht in Richtung des Pfeils II auf den expandierbaren Körper in gefalteter Form;
- Figur 3: einen Längsschnitt durch die Endoskopiereinrichtung, wobei der expandierbare Körper in die gewünschte Position vorgeschoben wurde;
- Figur 4: einen Längsschnitt durch die Endoskopiereinrichtung der Figur 3, wobei die Videoeinheit zurückgezogen wurde;
- Figur 5: einen Längsschnitt durch die Endoskopiereinrichtung der Figur 4, wobei das weitere Endoskop teilweise zurückgezogen wurde;
- Figur 6: einen Längsschnitt durch die Endoskopiereinrichtung der Figur 5, wobei das weitere Endoskop vollständig in das erste Endoskop zurückgezogen wurde und der expandierbare Körper freigegeben wurde;
- Figur 7: einen Längsschnitt durch eine Endoskopiereinrichtung gemäß der Darstellung der Figur 5, wobei zusätzlich ein weiteres Endoskop mit einer Greifvorrichtung parallel zu der Videoeinheit eingeführt worden ist und
- Figur 8: einen Längsschnitt durch die Endoskopiereinrichtung gemäß Figur 6, wobei zusätzlich ein weiteres Endoskop mit einer Greifvorrichtung das distale Ende des expandierbaren Körpers manipuliert.

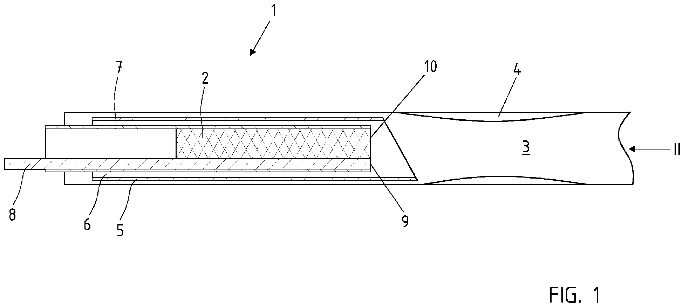

Die Figur 1 zeigt eine Endoskopiereinrichtung 1 zum Einbringen eines expandierbaren Körpers 2 (Stent) in einen Hohlraum 3. Der Hohlraum 3 weist eine Engstelle 4 auf. Im Bereich dieser Engstelle 4 soll der expandierbare Körper 2 mittels der Endoskopiereinrichtung 1 platziert werden. Hierzu wird ein erstes Endoskop 5 mit einem Arbeitskanal 6 in den Hohlraum 3 bis zu der gewünschten Tiefe eingeführt. Der expandierbare Körper 2 befindet sich in einem weiteren Endoskop 7, das in das erste Endoskop 5 eingeführt wird. In diesem weiteren Endoskop 7, von dem nur das Endoskoprohr dargestellt ist, befindet sich eine Videoeinheit 8 mit deutlich kleinerem Durchmesser. Die Videoeinheit 8 besitzt einen Durchmesser von beispielsweise 4 mm. Das weitere Endoskop 7 besitzt einen Durchmesser von 12 bis 14 mm. Die Videoeinheit 8 besitzt ein distales Ende 9, das sich am, d.h. unmittelbar neben dem distalen Ende 10 des expandierbaren Körpers 2 befindet. Der expandierbare Körper 2 befindet sich also parallel zu der Videoeinheit 8. Die Position des distalen Endes 9 der Videoeinheit 8 ist gleichzusetzen mit der Position der Kamera der Endoskopiereinrichtung 1, sodass unter Videokontrolle der expandierbare Körper 2 exakt bis hinter das distale Ende der Engstelle 4 eingebracht werden kann.

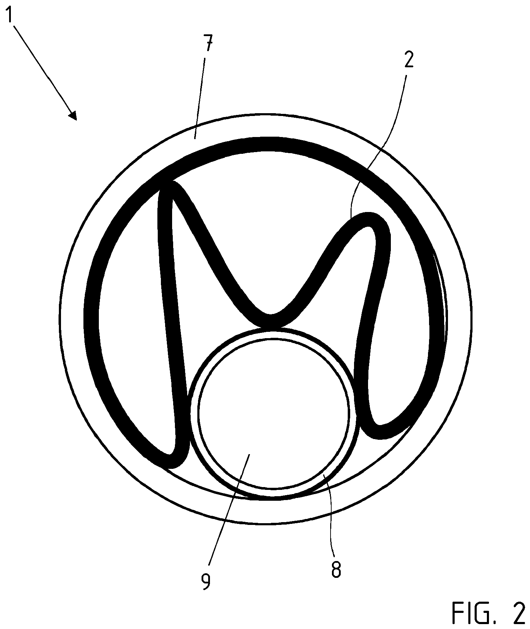

Die Figur 2 zeigt in einer Stirnansicht die Position des gefalteten, expandierbaren Körpers 2 in dem weiteren Endoskop 7 parallel zu der Videoeinheit 8 mit seinem distalen Ende 9, das gleichzeitig die Videokamera verkörpert. Das weitere Endoskop 7 besteht insbesondere aus PTFE oder weist eine innere Beschichtung aus PTFE aus.

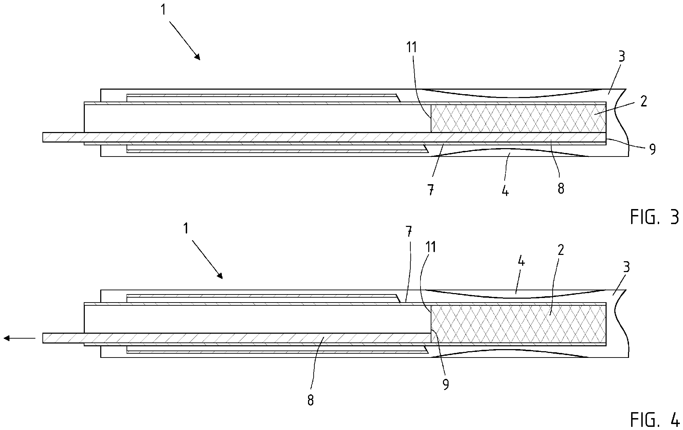

Die Figur 3 zeigt den nächsten Schritt des Einbringens des expandierbaren Körpers 2. Hierzu wird das weitere Endoskop 7 vorgeschoben, bis das distale Ende 9 der Videoeinheit 8 die Engstelle 4 im Hohlraum 3 passiert hat. Das weitere Endoskop 7 wird nun zurückgezogen, um den expandierbaren Körper 2 in dieser Position freizugeben. Hierzu bedarf es eines Widerlagers am distalen Ende 11 des expandierbaren Körpers 2.

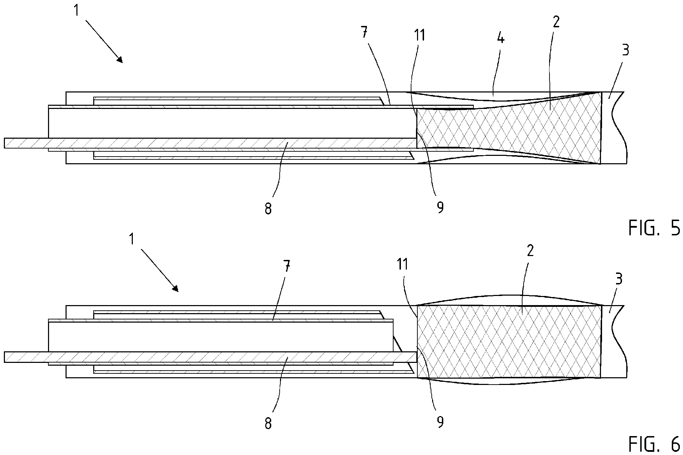

Das Widerlager wird dadurch geschaffen, dass die Videoeinheit 8 um die Länge des expandierbaren Körpers 2 zurückgezogen wird. Das zeigt die Figur 4. Nun befindet sich das distale Ende 9 der Videoeinheit 8 am proximalen Ende 11 des expandierbaren Körpers 2. In dieser Position wird die Videoeinheit 8 gehalten und das weitere Endoskop 7 zurückgezogen. Diesen Vorgang zeigen die Figuren 5 und 6 in zwei zeitlich aufeinander folgenden Schritten. In der Figur 5 ist der expandierbare Körper 2 nur teilweise freigegeben. In der Figur 6 ist der expandierbare Körper 2 vollständig freigegeben. Die Engstelle 4 wird von innen abgestützt. Der freie Durchgangsquerschnitt des Hohlraums 3 wurde vergrößert und es kann mittels der Videoeinheit 8 die korrekte Position des expandierbaren Körpers 2 kontrolliert werden.

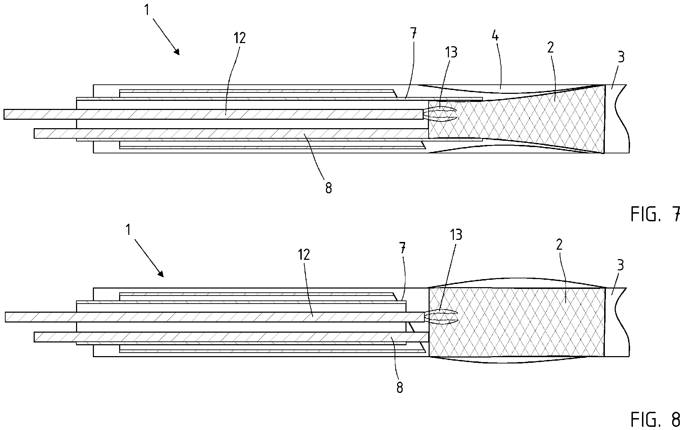

Da nicht alle expandierbaren Körper 2 zwangsläufig rotationssymmetrisch sind, kann die Notwendigkeit bestehen, den expandierbaren Körper 2 bspw. mit einer abgeflachten Seite in eine besondere Position zu drehen. Hierzu ist es möglich, ein weiteres Endoskop 12 mit einer Greifeinrichtung 13 zu verwenden, wie es in den Figuren 7 und 8 analog zu der zeitlichen Abfolge der Figuren 5 und 6 dargestellt ist. Mittels der Greifeinrichtung 13 kann der expandierbare Körper 2 um eine Längsachse gedreht werden. Es ist auch möglich, mittels des weiteren Endoskops 12 und der Greifvorrichtung 13 ein Widerlager für den expandierbaren Körper 2 zu schaffen. Das Videoendoskop 8 dient in diesem Fall nur zur Unterstützung bzw. kann zur visuellen Kontrolle auch zurückgezogen werden, wenn die Abstützung ausschließlich über das weitere Endoskop 12 mit der Greifvorrichtung 13 erfolgt. Das Ergebnis ist ein korrekt positionierter expandierbarer Körper 2 in dem Hohlraum 3.

### Bezugszeichen:

- 1 -: Endoskopiereinrichtung
- 2 -: expandierbarer Körper
- 3 -: Hohlraum
- 4 -: Engstelle in 3
- 5 -: Endoskop
- 6 -: Arbeitskanal von 5
- 7 -: weitere Endoskop
- 8 -: Videoeinheit
- 9 -: distales Ende von 8
- 10 -: distales Ende von 2
- 11 -: proximales Ende von 2
- 12 -: weiteres Endoskop
- 13 -: Greifvorrichtung

## Patentansprüche

1. Endoskopiereinrichtung (1) zum Einbringen eines expandierbaren Körpers (2) in einen Hohlraum (3), welche ein erstes Endoskop (5) mit einem Arbeitskanal (6) aufweist und ein weiteres Endoskop (7) aufweist, das sich in dem Arbeitskanal (6) befindet, wobei das weitere Endoskop (7) einen expandierbaren Körper (2) und eine Videoeinheit (8) aufweist, die sich parallel zu dem expandierbaren Körper (2) in dem weiteren Endoskop (7) befindet, mit folgenden Merkmalen:
a) in das erste Endoskop (5) mit dem Arbeitskanal (6) ist das weitere Endoskop (7) mit dem einzubringenden expandierbaren Körper (2) eingeführt;
b) ein distales Ende (9) der Videoeinheit (8) befindet sich neben einem distalen Ende (10) des expandierbaren Körpers (2), um den expandierbaren Körper (2) unter visueller Kontrolle in dem Hohlraum (3) vor dem Expandieren zu platzieren;
**dadurch gekennzeichnet, dass**
c) die Videoeinheit dazu ausgebildet ist, nach dem Positionieren des expandierbaren Körpers (2) zurückgezogen zu werden, um ein Widerlager an dem proximalen Ende (11) des expandierbaren Körpers (2) zu bilden, wenn das weitere Endoskop (7) zurückgezogen wird.

2. Endoskopiereinrichtung (1) nach Anspruch 1, **dadurch gekennzeichnet, dass** die Videoeinheit (8) flexibel ist.

3. Endoskopiereinrichtung (1) nach Anspruch 2, **dadurch gekennzeichnet, dass** die Flexibilität im Bereich des distalen Endes (9) der Videoeinheit (8) größer ist als an einem proximalen Ende der Videoeinheit (8).

## Claims

1. Endoscopy apparatus (1) for introducing an expandable body (2) into a cavity (3), said apparatus having a first endoscope (5) with a working channel (6) and having a further endoscope (7) which is located in the working channel (6), wherein the further endoscope (7) has an expandable body (2) and a video unit (8) which is located parallel to the expandable body (2) in the further endoscope (7), having the following features:
a) the further endoscope (7) with the expandable body (2) to be introduced is inserted into the first endoscope (5) with the working channel (6);
b) a distal end (9) of the video unit (8) is located next to a distal end (10) of the expandable body (2) in order to place the expandable body (2) under visual control in the cavity (3) prior to expansion;
**characterised in that**
c) the video unit is designed to be withdrawn after positioning of the expandable body (2) in order to form an abutment at the proximal end (11) of the expandable body (2) when the further endoscope (7) is withdrawn.

2. Endoscopy apparatus (1) according to claim 1, **characterised in that** the video unit (8) is flexible.

3. Endoscopy apparatus (1) according to claim 2, **characterised in that** the flexibility in the region of the distal end (9) of the video unit (8) is greater than at a proximal end of the video unit (8).

## Revendications

1. Équipement d'endoscopie (1) destiné à l'introduction d'un corps expansible (2) dans une cavité (3), équipement qui présente un premier endoscope (5) avec un canal de travail (6) et un autre endoscope (7) qui se trouve dans le canal de travail (6), dans lequel l'autre endoscope (7) présente un corps expansible (2) et une unité vidéo (8) qui se trouve dans l'autre endoscope (7) parallèlement au corps expansible (2), avec les caractéristiques suivantes :
a) l'autre endoscope (7) est inséré avec le corps expansible (2) à introduire dans le premier endoscope (5) avec le canal de travail (6) ;
b) une extrémité distale (9) de l'unité vidéo (8) se trouve à proximité d'une extrémité distale (10) du corps expansible (2) pour placer le corps expansible (2) sous contrôle visuel dans la cavité (3) avant l'expansion ;
**caractérisé en ce que**
c) l'unité vidéo est configurée pour être retirée après le positionnement du corps expansible (2) pour former un palier de butée au niveau de l'extrémité proximale (11) du corps expansible (2) lorsque l'autre endoscope (7) est retiré.

2. Équipement d'endoscopie (1) selon la revendication 1, **caractérisé en ce que** l'unité vidéo (8) est flexible.

3. Équipement d'endoscopie (1) selon la revendication 1, **caractérisé en ce que** la flexibilité dans la zone de l'extrémité distale (9) de l'unité vidéo (8) est plus grande qu'au niveau d'une extrémité proximale de l'unité vidéo (8).
